Europäisches Patentamt

European Patent Office

Office européen des brevets

(19)

(11) Publication number: **0 364 235**
**A1**

# (12) EUROPEAN PATENT APPLICATION

(21) Application number: 89310385.3

(22) Date of filing: 11.10.89

(51) Int. Cl.⁵: **A61K 37/02 , A61K 37/24 , A61K 37/26 , A61K 37/30 , A61K 9/14 , A61K 47/22**

(30) Priority: 14.10.88 JP 256919/88

(43) Date of publication of application:
18.04.90 Bulletin 90/16

(84) Designated Contracting States:
CH DE ES FR GB IT LI

(71) Applicant: TOYO JOZO KABUSHIKI KAISHA
632-1 Mifuku Ohito-cho
Tagata-gun Shizuoka-ken(JP)

(72) Inventor: Yamamoto, Nakayuki
90-5, Kashiya Kannami-cho
Tagata-gun Shizuoka(JP)
Inventor: Sugimoto, Michihiko
1-16, Satsuki-cho
Numazi-shi Shizuoka(JP)
Inventor: Sakakibara, Hideo
273-12, Naka
Mishima-shi Shizuoka(JP)

(74) Representative: Myerscough, Philip Boyd et al
J.A.Kemp & Co. 14, South Square Gray's Inn
London, WC1R 5EU(GB)

(54) Physiologically active peptide composition for nasal administration.

(57) A powder composition suitable for nasal administration comprising a physiologically active peptide as an active ingredient and a lactone of a water-soluble organic acid as an absorption promoter.

EP 0 364 235 A1

## PHYSIOLOGICALLY ACTIVE PEPTIDE COMPOSITION FOR NASAL ADMINISTRATION

This invention relates to a nasal administration powder composition containing a physiologically active peptide as an active ingredient which has been improved in efficiency of abosorption through nasal mucosa.

Peptide hormones such as insulin and calcitonin which are used as clinical drugs at present are susceptible to hydrolysis with enzymes in gastrointestinal tracts and on wall of gastrointestinal tracts and are very difficult to have them absorbed through gastrointestinal tracts. Thus, hitherto, they have been administrated only by injections.

However, injections often cause pains and are not preferred and other administration methods have been attempted. According to the various studies, there have been proposed a preferable route of a nasal administration for peptide hormones. However, none have not yet been put to practical use because of lower absorption rate than by injections and addition of absorption promoter has been required.

Some attempts have been made on nasal administration and, for example, a method is known which uses a surface active agent as an absorption promoter with insulin or calcitonin [e.g., Japanese Patent Kokai Nos. 59 - 89619 and 59 - 130820, J. Pharm. 9: 165 - 172 (1981), Proc. Natl. Acad. Sci. USA, 82:7419-7423 (1985), J. Pharm. Sci., 76: 351 - 355 (1987), J. Pharmacobio-Dyn., 10: 624 - 631 (1987), Pharm. Res. , 5: 305 - 308 (1988) and Diabetes, 27: 296 - 299 (1978))] . However, the drug is liquid and readily runs out after administration. There are further problems that safety on nasal mucosa and stability of drug are damaged due to addition of the surface active agents and disinfectant.

On the other hand, powdered nasal administration preparations have been proposed. Powdered nasal administration preparation was already put to practical use as preparation for Intal (tradename, Fujisawa Pharmaceutical Co., Ltd., FISONS plc England) in 1975. Further powdered composition for nasal administration containing water absorbing base was proposed as peptide preparation (Japanese Patent Kokai No. 59 - 163313). However, this preparation was practically not necessarily excellent since the effective component is not sufficiently absorbed from nasal cavity.

The object of this invention is to provide a powdered nasal administration composition contaning peptide hormone as an active ingredient which is superior in safety and stability and from which the active ingredient can be fully absorbed through nasal cavity.

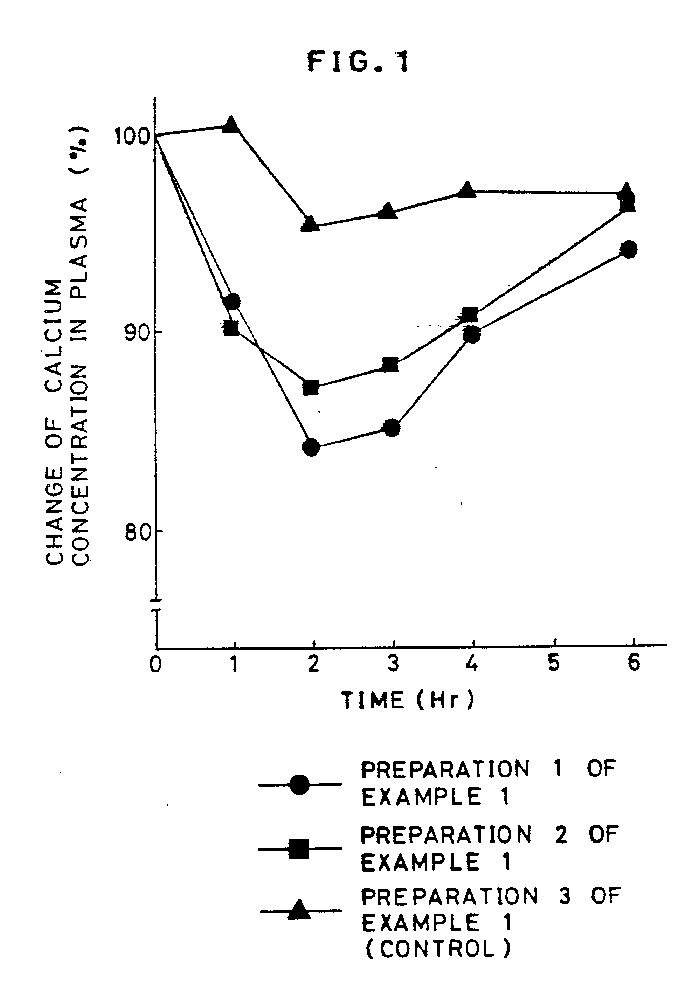

Fig. 1 is a graph which shows changes in calcium concentration inplasma of rabbits when preparations 1 and 2 in Example 1 of this invention and preparation 3 containing no lactone compound as a control were administered to rabbits, respectively.

The inventors already obtained a powdered preparation containing a water-soluble organic acid as an absorption promoter is superior in absorption as a nasal administration composition (Japan Patent Appln. No. 63 - 144704).

As a result of further studies on a nasal administration composition containing physiologically active peptides such as calcitonins or insulin, we have found that powdered preparations for nasal administration containing physiologically active peptide with additionally adding a lactone compound of water-soluble organic acid as an adsorption on promoter showed excellent absorbability and stability. The lactone compound of organic acid is estimated to generatean organic acid by gradual hydrolysis in an aqueous liquid forabsorption stimulant, however there have no clear reasons.

That is, this invention relates to a nasal administration powdered composition containing a physiologically active peptide as an active ingredient, characterized by containing a lactone compound of water-soluble organic acid as an absorption promoter and, if necessary, a diluent.

The physiologically active peptides which are active ingredients in the composition of this invention include, for example, peptide hormones, proteins and enzymes which have physiological activity such as calcitonin, parathyroid hormone (PTH), calcitonin gene related peptides (CGRP), insulin, somatostatin, growth hormone, secretin, gastrin, vasopressin, oxytocin, glucagon, adrenocorticotropic hormone (ACTH), tyroid-stimulating hormone (THS), prolactin, luteinizing hormone-releasing hormone (LH-RH), endorphin, enkephalin, neurotensin, cytokine for example lymphokine or monokine such as interferon interleukin, TNF and CSF, enzymes such as superoxide dismutase and glutathione peroxidase and derivatives or modified peptide analogues in which one or more amino acid in a normal or natural type of peptides is modified by chemical means or genetic engineering thechnique and slats thereof. Besides, known peptide hormones, proteinsand enzymes having a molecular weight of up to about 30,000 are also included.

Among the above physiologically active peptides, preferred are peptides or derivatives thereof having a molecular weight whithin the range of 1,000 - 10,000. More preferred are calcitonins, parathyroid hormones (PTH) and insulins.

The calcitonins may be peptides having hypocalcemic activity and include various natural calcitonins

2

and pharmaceutically active derivatives thereof. Examples of the natural calcitonins are eel calcitonin, human calcitonin, salmon calcitonin, porcine calcitonin and chiken calcitonin. Examples of the pharmaceutically active derivatives thereof are [ASU$^{1,7}$] eel calcitonin (WHO generic name: elcatonin), [AUS$^{1,7}$] salmon calcitonin, [ASU$^{1,7}$] human calcitonin and [ASU$^{1,7}$] chicken calcitonin. Especially preferred is elcatonin.

These substances can be prepared by the processes disclosed, for example, in British Patent No. 1,516,947 and preliminary manuscript collection of lecture II, page 947 for the 50th spring meeting of Japan Chemical Society, in 1985. Moreover, other calcitonin-like peptides having hypocalcemic activity can be used in this invention. The calcitonin participates in disorder of bones, endocrine and digestive organs and are used for treatment of hypercalcemia, pains in osteoporosis and Paget's disease.

Concentration of calcitonin in the composition of this invention is normally 0.1 U/mg - 100 U/mg, preferably 1 U/mg - 50 U/mg. Dosage is preferably 10 - 50 mg/administration and number of administration is suitably 1 - 3 times a day.

The PTH are peptides having hypercalcemic activity and 34 - 84 amino acid sequences and include natural PTH and pharmaceutically active derivatives thereof. As examples thereof, mention may be made of human- PTH (h-PTH)(1 - 84) [Biochemistry 17, 5723 (1978) ] , h-PTH (1 - 38) [Japanese Patent Kokai No. 57 - 81448 ] , h-PTH (1 - 34) [Hoppe-Seyler's Z. Physiol. Chem., 355, 415 (1974) ] , h-PTH (1 - 34) NH$_2$ - [Japanese Patent Kokai No. 58 - 96052 ] , (Nle$^{8,18}$ ) h-PTH (1 - 34), [Nle$^{8,18}$, Tyr$^{34}$] h-PTH (1 - 34) [Japanese Patent Kokai No. 55 - 113753 ] ,. [Nle$^{8,18}$ ] h-PTH (1 - 34) NH$_2$ [Ja panese Patent Kokai No. 61 - 24598] , [Nle$^{8,18}$, Tyr$^{34}$] h-PTH (1 - 34) NH$_2$ [Japanese Patent Kokai No. 60-34996] , rat-PTH (1 - 84) [J. Biol. Chem., 259 (5), 3320 (1984)] , rat-PTH (1 - 34) [Endocrinol., 117 (3), 1230 (1985) ] , bovine-PTH (1 - 84) [Am. J. Med., 50, 639 (1971) ] , bovine-PTH (1 - 34), bovine- PTH (1 - 34) NH$_2$ [Pthobiology annual 11, 53 (1981), etc.

Insulines includ human insulin, porcine insulin, bovine insulin, horse insulin and sheep insulin.

As the lactone compound of water-soluble organic acids used in this invention, mention may be made of, for example, a mixture of one or more aldonolactone compound such as gluconolactone or glucuronolactone (glucurone or glucuronic acid lactone).

In this invention, diluents are used, if necessary. The diluents used in this invention are water-soluble or sparingly soluble and examples thereof are saccharoses, polysaccharides, dextrins, celluloses, synthetic or semisynthetic polymers, amino acids, polyamino acids, proteins nucleic acids, phospholipids and lipids.

The saccharoses (monosaccharides, oligosaccharides) include, for example, D-mannitol, glucose, lactose, fructose, inositol and sucrose. The polysaccharides include, for example, dextran, pullulan, alginic acid, hyaluronic acid, pectic acid, phytic acid and phytin. The dextrins include, for example, α-cyclodextrin, β-cyclodextrin, γ-cyclodextrin, dextrin, hydroxypropyl starch and hydroxyethyl starch.
The celluloses include, for example, methyl cellulose, ethyl cellulose, hydroxyethyl cellulose, hydroxypropyl cellulose, hydroxypropylmethyl cellulose and sodium carboxymethyl cellulose.

The synthetic and semisynthetic polymers include, for example, polyvinyl alcohol, carboxyvinyl polymer, polyethylene glycol, polyvinylpyrolidone (PVP), sodium polyacrylate and polylactic acid.

The amino acids include, for example, glycine and taurine. The polyamino acids include, for example, polyglutamic acid, polyaspartic acid, polyglycine and polyleucine.

The proteins include, for example, gelatin. Further, chitin and chitosan canbe mentioned.

Of these diluents, especially preferred are α-cyclodextrin, β-cyclodextrin, dextrin, D-mannitol, inositol, lactose, dextran, methyl cellulose, hydroxypropyl cellulose, polyvinyl alcohol and pullulan.

The proportion of the components in the powdered composition of this invention varies depending on the kind of the component, but normally is physiologically active peptide: about 0.005 - 20 % by weight, preferably about 0.01 - 10 % by weight, lactone of water-soluble organic acid: about 0.05 -99.95 % by weight, preferably about 0.5 - 99.99 % by weight and diluent: about 0.05 - 99.5 % by weight which is added, if necessary. The lactone of water-soluble organic acid is at least in such an amount that aqeuous solution of the powdered composition is acidic and may be optionally determined depending on clinical uses.

In addition, additives required for formulation of the powdered preparation can be added, if necessary.
The nasal administration powdered composition of this invention can be prepared by known methods.

For instance, lactone of water-soluble organic acid and, if necessary, diluent are to peptide, followed by mixing. Alternatively, a mixture of peptide and diluent is once dissolved in a distilled water, then lyophilized. Thereafter, the mixture was ground and a lactone of water-soluble organic acid or a lactone of water-soluble organic acid and diluent is added to obtain a homogeneous nasal administration powdered composition. Alternatively, peptide and lactone of water-soluble organic acid and, if necessary, diluent are once dissolved in a solvent such as distilled water and lyophilized and then ground to obtain the powdered composition. Or, peptide and lactone of water-soluble organic acid are once dissolved in a distilled water and lyophilized and then ground to powder. To the resulting lyophilized powder is added diluent or are added lactone of water-soluble organic acid and diluent, and these are mixed to obtain a homogeneous composition.

The resulting powdered composition is superior in solubility and so the particle size of the components is not critical, but prepferably 80 % or more of the composition have 300 microns or less. Especially, if 80 % or more is dispersed in the range of 1 - 200 microns, durability can be somewhat improved.

The powdered composition of this invention is used, in any preparation which can be administered nasally. For example, a sealed type nasal administration powdered compopsition with jet-sparay of $CO_2$ or freon gas, or aerosol type nasal administration composition suspended in liquid freon can be mentioned. Further composition can be filled in a capsule. This capsule is fixed in a spraying container for nasal administration and small holes are pricked by a needle at both ends of the capsule on use. Then, air is blown thereinto to jet the powdered composition into nasal cavity. However, method of the administration is not critical.

The nasal administration powdered composition of this invention is superior to the conventional liquid preparations for nasal administration of peptide hormone in stability of active ingredient. Further, in the conventional liquid preparations for nasal administration of peptide hormone, surface active agents are used as absorption promoter, which are highly irritative against nasal mucosa and bisides, preservatives are used for preventing contamination with microorganisms, which cause harmful effects. On the other hand, preparations comprising the nasal administration powdered composition of the present invention suffer from no such problems.

In addition, the nasal administration powdered composition of the present invention is much superior to the conventional nasal administration powdered preparations in absorbability through nasal mucosa.

The following examples and experimental examples illustrate the present invention in more detail but are not construed as limiting.

Example 1

(a) 50,000 U of elcatonin ( [Asu$^{1.7}$] eel calcitonin, 6,000 U/mg) and 489.6 mg of D-mannitol were taken in a beaker and 25 mℓ of distilled water was added thereto to dissolve them. The solution was lyophilized and ground in a mortar to obtain uniform lyophilized powder containing 100 U/mg of elcatonin.

Preparation 1

Then, 20 mg of the resulting lyophilized powder containing 100 U/mg of elcatonin obtained in the above (a) and 200 mg of glucono delta lactone (Sigma Chem., Co.) were taken in a mortar and well mixed and thereto was added gradually 780 mg of dextran (Sigma. Chem. Co., an average molecular weight of 40,200) with mixing to obtain a uniform powdered preparation. The resulting powdered preparation 2 U/mg of elcatonin.

Preparation 2

20 mg of the lyophilized powder containing 100 U/mg of elcatonin obtained in (a) hereinabove and 200 mg of D-glucurono lactone were taken in a mortar and well mixed and thereto was gradually added 780 mg of dextran (Shigma Chem. Co., an average molecular weight of 40,200) with mixing to obtain a uniform powdered preparation. The resulting powder preparation contained 2 U/mg of elcatonin.

Preparation 3 (for control)

20 mg of the lyophilized powder obtained in (a) hereinabove containing 100 U/mg of elcatonin was taken in a mortar and thereto was gradually added 980 mg of dextran (Sigma Chem. Co., an average molecular weight of 40,200) with mixing to obtain a uniform powdered preparation which was a control containing no glucono delta lactone as a absorption promoter for comparison with the preparation of the present invention. The resulting powdered preparation contained 2 U/mg of elcatonin.

Experimental Example 1

(a) Experiment for absorption of an elcatonin nasal administration preparation through nasal mucosa in rabbits.

To Japanese White strain male rabbits (body weight: about 3 kg, 5 rabbits in one group) which had been fasted overnight and anesthetized were administered nasally the preparations 1 and 2 (5 U/2.5 mg/kg) prepared in Example 1. To the rabbits was administrated similarly preparation 3 (control) prepared in Example 1 for comparison.

2 mℓ of blood was taken from an ear vein before the administration and at 1, 2, 3, 4 and 6 hours after the administration. These specimens were centrifuged at 3000 r.p.m. for 10 minitus to obtain plasma. Calcium concentration in plasma was determined by an atomic absorptiometer.

(b) Results

After administration of elcatonin nasal administration preparation, calcium concentration in plasma was measured to examine absorption of elcatonin through nasal mucosa. The results are shown in Fig. 1. As clearly administration of the preparations of the present invention revealed marked reduction of calcium concentration in plasma as compared with the control preparation 3. The results indicate that absorptions of the present through nasal mucosa is markedly enhanced by the addition of glucono delta lactone or D-glucurono lactone as an absorption promoter.

Example 2

20,000 units of elcatonin and 1.0 g of fine powdered glucono delta lactone were put in a mortar and were well mixed to obtain a uniform powdered preparation containing 20 U/mg of elcatonin.

Example 3

4,000 units of salmon calcitonin (4,000 U/mg) and 100 mg of glucono delta lactone were dissolved in 10 mℓ distilled water and immediately lyophilized and gorund in mortar to obtain powdered preparation containing 40 U/mg of salmon calcitonin.

Example 4

20,000 units of h-PTH (1 - 34) and 1.0 g of the previously finely ground glucono delta lactone were taken in a mortar and well mixed to obtain a powdered preparation contaning 20 U/mg og h-PTH (1 - 34).

Example 5

10,000 units of h-PTH (1 - 34) and 1.0 g of glucono delta lactone were taken in a mortar and well mixed to obtained a powdered preparation containing 10 U/mg of h-PTH (1 - 34).

Example 6

Preparation 4

100 mg of porcine insulin sodium salt (CALBIOCHEM, tradename, manufactured by Calbiochem, lyophilized specific activity: 26.3 U/mg, water content: 9.88%) and 100 mg of glucono delta lactone were taken in a mortar and well mixed and then mixed with gradual addition of 800 mg of dextran (average molecular weight: 40,200, Sigma Chem. Co.) as a diluent to obtain a uniform powder preparation. The resulting powder preparation contained 2.37 U/mg of insulin.

Preparation 5 (for control)

100 mg of porcine insulin sodium salt (CALBIOCHEM, tradename, manufactured by Calbiochem, lyophilized specific activity: 26.3 U/mg, water content: 9.88%) and 900 mg of dextran (average molecular weight: 40,200, Sigma Chem. Co.) were taken in a moltar and well mixed to obtain a uniform powder preparation containng no lactone of organic acid as a control preparation comparison.

Experimental Example 2

(a) Experiment for nasal administration of an insulin nasal administration preparation in rabbits .

To Japanese White strain male rabbits (body weight: about 3kg, 5 rabittes in one goupe) which had been fasted for 17 hours before administration and anesthetized with pentobarbital (25 mg/kg) were administered nasally 5 U/2.1 mg kg of the preparation 4 of Example 5 or the preparation 5 (control) obtained in Example 5.

2 m$l$ of blood was taken from an ear vein 5 minutes before the administration and 30 minutes, 1 hour,2 hours, 3 hours, 4 hours and 6 hours after the administration. These blood sample were centrifuged at 3,000 r.p.m. for 10 minutes to obtain plasma.

Evalution on absorbility of the insulin preparation through nasal mucosa was performed by measuring decrease of glucose concentration in plasma. The glucose concentration in plasma was measured by using a kit of clinical test chemicals Glucose-Test Wako (Wako Junyaku Co).

The glucose concentration in the plasma obtained for the blood taken before the administration was shown as the standard value (100 %) for glucose concentration in plasma.

(b) Results

The results are shown in Table 1.

Table 1

| Example | ratio of changes glucose concnetration in plasma | | | | | | |
|---|---|---|---|---|---|---|---|
| | 0 | 0.5 hr | 1 hr | 2 hr | 3 hr | 4 hr | 6 hr |
| preparation 4 (the present invention) | 100 | 59.8 | 53.5 | 44.1 | 55.1 | 63.8 | 85.8 |
| preparation 5 (control) | 100 | 106.5 | 102.3 | 100.0 | 100.9 | 102.3 | 95.3 |

The results shown in Table 1 indicate that insulin was more efficiently absorbed through the nasal mucosa by addition of lactone of organic acid as an absorption promoter as compared with the control preparation.

**Claims**

1. A powder composition suitable for nasal administration comprising a physiologically active peptide as an active ingredient and a lactone of a water-soluble organic acid as an absorption promoter.

2. A composition according to claim 1 wherein the physiologically active peptide is a peptide or derivative thereof having a molecular weight of from 1000 to 10,000.

3. A composition according to claim 1 or 2 wherein the lactone of a water-soluble organic acid is an aldonolactone.

6

4. A composition according to claim 3 wherein the aldonolactone is gluconolactone or glucuronolactone.

5. A composition according to any one of the preceding claims which additionally comprises a diluent.

6. A composition according to claim 5 wherein the diluent is at least one selected from saccharoses, polysaccharides, dextrins, celluloses, synthetic or semisynthetic polymers, amino acids, polyamino acids, proteins, nucleic acids and lipids.

7. A composition according to claim 6 wherein the diluent is mannitol and/or dextran.

8. A composition according to any one of the preceding claims wherein the physiologically active peptide is selected from calcitonins, parathyroid hormones and insulin.

9. A composition as defined in any one of the preceding claims for use in a method of treatment of the human or animal body by therapy.

10. A process for preparing a composition as defined in any one of claims 1 to 8 which comprises mixing together the physiologically active peptide, the lactone of a water-soluble organic acid and, if desired, the diluent.

# FIG. 1

PREPARATION 1 OF EXAMPLE 1

PREPARATION 2 OF EXAMPLE 1

PREPARATION 3 OF EXAMPLE 1 (CONTROL)

| | DOCUMENTS CONSIDERED TO BE RELEVANT | | EP 89310385.3 |
|---|---|---|---|

| Category | Citation of document with indication, where appropriate, of relevant passages | Relevant to claim | CLASSIFICATION OF THE APPLICATION (Int. Cl.5) |
|---|---|---|---|
| X | WO - A1 - 87/03 473 (D.HSIEH) * Abstract; claims 10,13, 26-29; page 5, line 1 - page 6, line 14; page 22, line 1 - page 27, line 10 * -- | 1,2,5, 6,8-10 | A 61 K 37/02 A 61 K 37/24 A 61 K 37/26 A 61 K 37/30 A 61 K 9/14 A 61 K 47/22 |
| Y | EP - A1 - 0 122 036 (TEIJIN LIMITED) * Abstract; claims 1,2,11-14; page 4, line 25 - page 5, line 24 * -- | 1-6,8-10 | |
| Y | EP - A2 - 0 067 513 (TAKEDA CHEM. IND. LTD.) * Claims 1,6; page 6, lines 5-12 * -- | 1-6,8-10 | |
| Y | EP - A2 - 0 273 202 (VAN SCOTT, R.J. YU) * Abstract; claims 1,4,7,9, 12,34-36 * -- | 1-6,8-10 | TECHNICAL FIELDS SEARCHED (Int. Cl.4) |
| A | EP - A2 - 0 193 372 (TEIJIN LTD.) -- | | A 61 K 37/00 A 61 K 9/00 A 61 K 47/00 |
| A | US - A - 4 476 116 (T.A.SHABIR) -- | | |
| A | EP - A2 - 0 183 527 (YAMANOUCHI PHARM. CO. LTD.) -- | | |
| A | EP - A2 - 0 242 643 (DELAWARE CHEM. CORP.) -- | | |
| A | DE - A1 - 3 424 057 | | |

The present search report has been drawn up for all claims

| Place of search | Date of completion of the search | Examiner |
|---|---|---|
| VIENNA | 22-12-1989 | MAZZUCCO |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| | (WITTO ELECTRIC IND. CO.)<br><br>---- | | |

**TECHNICAL FIELDS SEARCHED (Int. Cl.4)**

The present search report has been drawn up for all claims

| Place of search | Date of completion of the search | Examiner |
|---|---|---|
| VIENNA | 22-12-1989 | MAZZUCCO |